# EUROPEAN PATENT SPECIFICATION

(11) **EP 2 276 434 B1**
(45) Date of publication and mention of the grant of the patent: **15.02.2012**
(21) Application number: 08873945.3
(22) Date of filing: 18.12.2008
(51) Int. Cl.: A61L 28/00, A61L 24/04, A61L 24/00, A61F 5/443, A61F 5/445

(54) **A COLLECTING DEVICE FOR BODY FLUIDS**
VORRICHTUNG ZUM AUFFANGEN VON KÖRPERFLÜSSIGKEITEN
DISPOSITIF COLLECTEUR DE FLUIDES CORPORELS

(30) Priority: 17.04.2008 DK 200800561; 25.04.2008 DK 200800589
(43) Date of publication of application: 26.01.2011
(73) Proprietor: Coloplast A/S, 3050 Humlebæk (DK)
(72) Inventor: STROEBECH, Esben, DK-2970 Hoersholm (DK); BACH, Anders, DK-2300 Koebenhavn S (DK)
(86) International application number: PCT/DK2008/050317
(87) International publication number: WO 2009/127207

(56) References cited:
- EP-A- 0 413 250
- EP-A- 0 686 381
- WO-A-2005/102229
- WO-A-2007/128320
- WO-A-2008/154928

## Description

### Field of the Invention

The present invention relates to a collecting device for attachment to human skin and for collecting bodily waste e.g. an ostomy device.

### Background

Collecting devices for collecting bodily waste, ostomy appliances, wound or fistulae drainage bandages or devices for collecting urine are usually in the form of a receptacle, e.g. a bag, pouch or tube for receiving the waste, connected to an adhesive wafer that can be attached to the skin of the patient. The wafer is typically in the form of a backing layer coated on the skin-facing surface with an adhesive layer and the wafer may further be provided with an aperture for accommodating the body opening. The size and shape of said aperture can often be adapted individually to fit the anatomy of the patient.

One of the crucial part of such devices is the adhesive wafer. The wafer should be able to fit leak proof around the body opening, have good adherence to the skin without unintended detachment from the skin, but at the same time be easy to remove again without damaging the skin. Furthermore, the wafer should be able to follow the movements of the body and be comfortable to wear.

When designing a skin adhesive one of the major issues is to keep the skin relatively dry underneath the adhesive to prevent maceration. Maceration occurs when the skin is unable to get rid of moisture from transpiration and outlet from a body opening. This may result in degradation of the skin's barrier function as well as bad adhesion of the device to the skin.

Usually, skin adhesive keeps the skin dry by absorbing moisture. Absorbing particles or hydrocolloids (HC) are mixed into an adhesive matrix in order to absorb moisture from the skin and thereby the skin is kept relatively dry. This technique is well known in the art and forms the basis for most ostomy adhesives that are commercially available see, e.g. US Patent No. 4,192,785.

To improve the ability of the adhesive wafer to resist leakage, some wafers include a secondary adhesive surrounding a first adhesive center. The first adhesive is positioned adjacent to the output source and the secondary adhesive supplies additional leakage resistance. US Patent No. 4,775,374 describes an adhesive construction, wherein both the first and the secondary adhesive are hydrocolloid (HC) based. In this adhesive construction, both the first and the second adhesive are sensitive to the output that they are supposed to resist. Additionally, they will both swell when getting in contact with the output. Thus, the failure mechanism is the same for both adhesives and the added protection is limited. Other products, such as the Microskin adhesive with washer sold by Cymed, include a first and a second adhesive where the second outer adhesive is an acrylic adhesive. Acrylic adhesives are known to cause allergic reactions on skin and are therefore problematic. Furthermore, known secondary adhesives strip or otherwise damage the skin upon removal, which can be painful and discomforting to the user.

D1 (EP-A-0686381) discloses an ostomy appliance in which the faceplate assembly includes two layers of hydrocolloid-containing adhesive barrier materials of different compositions.

D2 (WO 2005/102229 A) discloses an ostomy appliance comprising an ostomy bag and means for attaching the ostomy bag to a user's skin, the attaching means comprising a radially inner layer which provides cushioning and also some degree of adhesion to the user's skin, and a radially outer layer of thin flexible oxygen and water vapour permeable but water and body fluids impermeable material which surround the inner layer and which provides stronger adhesion to the user's skin than the inner layer.

D3 (EP-A-0413250) discloses occlusive attaching means comprising a first relatively thick adhesive component to be secured to the skin around the stoma and a relatively thin flexible second adhesive component to be secured to the skin at a distance from the stoma. Both adhesive components include an occlusive adhesive layer formulated as a blend containing one or more water soluble or swellable hydrocolloids dispersed in polyisobutylene along with other optional ingredients.

It has now surprisingly been found that by combining a standard HC adhesive with a polyalkyleneoxide polymer and an organosiloxane based cross-linked adhesive system, the problem with allergic reactions can be avoided. Furthermore, the standard HC adhesive and the cross-linked adhesive system are very different in adhesion properties. Thus, the two adhesives characteristics can complement each other to achieve better adhesion than one of the adhesives could achieve alone. Finally, the polyalkyleneoxide polymer and an organosiloxane based cross-linked adhesive system is non-damaging and less painful to remove, providing a more comfortable secondary adhesive.

### Summary of the invention

The present invention relates to a body waste collecting device comprising a collecting pouch and an adhesive wafer for attachment to the body, said adhesive wafer comprises a backing layer, a first adhesive and an additional second adhesive, wherein said additional second adhesive comprises a polyalkyleneoxide polymer and an organosiloxane based cross-linked adhesive system and said first adhesive is a hydrocolloid pressure sensitive adhesive.

### Brief description of the drawing

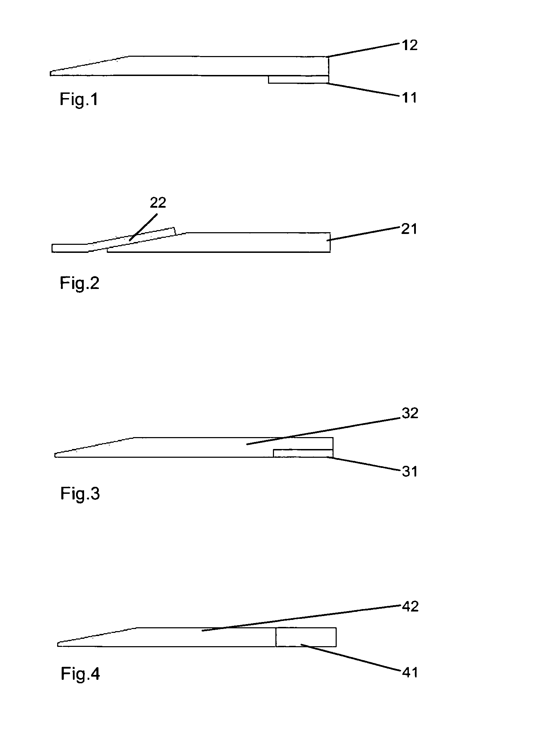

The invention is disclosed more in detail with reference to the drawings in which Figures 1 to 4 show different embodiments of the invention.

### Detailed description of the present invention

The aim of the invention is to provide a body waste collecting device that includes a first adhesive and an additional second adhesive, wherein said additional second adhesive comprises a polyalkyleneoxide polymer and an organosiloxane based cross-linked adhesive system.

By body waste collecting device is meant a device being able to collect and hold the output in a collecting item for a predefined time. The holding in place of the device may be obtained by a skin adhesive and the collection may be obtained by a bag.

In an embodiment of the invention a body waste collecting device comprises a collecting pouch and an adhesive wafer for attachment to the body, said adhesive wafer comprises a backing layer, a first adhesive and an additional second adhesive, wherein said additional second adhesive comprises a polyalkyleneoxide polymer and an organosiloxane based cross-linked adhesive system.

The polyalkyleneoxide polymer and the organosiloxane based cross-linked adhesive system does not absorb the moisture but rather permeate the water away from the skin surface. Thus, the swelling effect caused by the hydrocolloids will usually not occur. The adhesion is usually obtained by wetting and affinity to the skin.

The additional second adhesive is used for security and protection for the user in order to avoid leakages and to obtain longer wear time.

According to an embodiment of the invention, the first adhesive is located adjacent to the body waste source, usually an ostomy or a fistula.

By located adjacent to is meant that the subject is located close to or even in contact with the other subject.

In one embodiment of the invention, the first adhesive is a hydrocolloid pressure sensitive adhesive.

A standard and conventional hydrocolloid (HC) pressure sensitive adhesive is a skin friendly adhesive that is capable of adhering to the skin, handling perspiration by absorbing the moist and being removable from the skin without essential damage.

A typical pressure sensitive adhesive is based on a polyisobutylene, PIB (adhering substance) and carboxy methyl cellulose, CMC (absorbing media).

A typical HC pressure sensitive adhesive as used today is a highly filled hydrocolloid system with a matrix of polymers that exhibit flow as well as cohesive properties. The hydrocolloids absorb the moisture from perspiration and output from the body and the polymers adhere to the skin through skin polymer affinity and the polymers flow into the small cavities of the skin. The cohesion of the adhesive ensures that erosion is minimised and that the wafer is removed in one piece and that minimal adhesive residues are left at the skin.

The collecting device according to the invention combines the mechanical and adhesive properties of the two kinds of known skin adhesives as described above. A negative effect of the swelling of the hydrocolloid-based skin adhesive is used according to the invention in a beneficial way to provide a sealing effect. In the use of an ostomy appliance the requirements from the adhesive in the peri stomal area are often different as the use varies within stoma type, body shape, user patterns etc.

When using a hydrocolloid adhesive as the first adhesive according to the invention, the peri stomal area of the adhesive wafer tends to be stiffer resulting in a fixation of the skin in the inner part of the wafer and a more soft and flexible part in the outer zone. The slightly stiffer inner zone is typically obtained from the high filler rate of absorbing particles and choice of adhesive materials that are stiffer than the additional second adhesive.

Such an embodiment according to the invention is especially beneficial when used in cases where very liquid or skin aggressive output is exposed to the inner hole of the adhesive wafer for a long time. A typical example is the use of a 2-piece appliance for ileo ostomy where the adhesive wafer stays on the skin for several days.

The additional second adhesive of the invention comprises a polyalkyleneoxide polymer and an organosiloxane based cross-linked adhesive system.

As used herein a cross-link means a small region in a macromolecule (polymer chain structure) from which more than 2 chains emanate.

According to one embodiment of the invention, the additional second adhesive comprises the reaction product of:
(i) a polyalkyleneoxide polymer having one or more unsaturated end groups and
(ii) an organosiloxane comprising one or more Si-H groups, carried out in the presence of an addition reaction catalyst.

According to another embodiment of the invention, the additional second adhesive comprises more than 90 % w/w of the polyalkylene oxide polymer that consists of polymerised alkyleneoxide moities having three or more carbon atoms.

According to another embodiment of the invention, the additional second adhesive comprises the reaction product of:
(i) a polyalkyleneoxide polymer having at least two unsaturated end groups and wherein more than 90 % w/w of the polyalkylene oxide polymer consists of polymerised alkyleneoxide moities having three or more carbon atoms,
(ii) a polysiloxane cross-linking agent comprising 3 or more Si-H groups and optionally
(iii) a polysiloxane chain extender comprising up to 2 Si-H groups carried out in the presence of an addition reaction catalyst.

According to a preferred embodiment of the invention, the addition reaction catalyst is a Pt vinyl siloxane complex.

According to a further preferred embodiment of the invention, the weight percent of polyalkylene oxide in said reaction product is 60 % or above.

The polyalkylene oxide polymer having one or more unsaturated groups may be branched or linear.

However, suitably, the polyalkylene oxide polymer is linear and has two unsaturated end groups.

In one particular embodiment of the invention, the polyalkylene oxide polymer is polypropyleneoxide.

The polypropylene oxide having unsaturated end groups may be a compound of formula

CH₂=C(R¹)-(Z)-O-(X)ₙ-(W)-C(R²)=CH₂ (Ia)

or

CH(R¹)=CH-(Z)-O-(X)ₙ-(W)-CH=CH(R²) (Ib)

wherein R¹ and R² are independently selected from hydrogen and C₁₋₆-alkyl;
Z and W is C₁₋₄-alkylene;
X is -(CH₂)₃-O- or - CH₂-CH(CH₃)-O-; and
n is 1 - 900, more preferred 10 - 600, or most preferred 20 - 600.

The number average molecular weight of the polyalkylene oxide having unsaturated end groups is suitably between 500 and 100,000, more preferred between 500 and 50,000 and most preferred between 1,000 and 35,000.

Polypropylene oxide having unsaturated end groups may be prepared as described in US Patent No. 6.248.915 and International Publication No. WO 05/032401 or analogously to the methods described therein. Other polyalkylene oxide polymers may be prepared analogously.

The polysiloxane cross-linking agent comprising 3 or more Si-H groups is suitable a compound having the formula

R-SiO(R, R)-(SiO(R,R))ₘ-Si-(R,R,R) (II)

wherein at least three of the groups R are hydrogen and the rest of the groups R are each independently selected from C₁₋₁₂-alkyl, C₃₋₈-cycloalkyl, C₆₋₁₄-aryl, and C₇₋₁₂-arylalkyl; and m is 5 to 50, or preferably 10 to 40. The number average molecular weight as determined by GPC is suitably 500-3,000.

One or more cross-linking agents of formula (II) may be used in the cross-linking reaction.

In one embodiment of the invention, a mixture of one or more cross-linking agents of formula (II) comprising 3 or more Si-H groups and a polysiloxane chain extender comprising up to 2 Si-H groups is used in the cross-linking reaction.

The polysiloxane chain extender is suitably a compound having the formula

R³-SiO(R³, R³)-(SiO(R³,R³))ₘ-Si-(R³,R³,R³) (III)

wherein up to 2 of the groups R³ are hydrogen and the rest of the groups R³ are each independently selected from C₁₋₁₂-alkyl, C₃₋₈-cycloalkyl, C₆₋₁₄-aryl, and C₇₋₁₂-arylalkyl; and m is 0 to 50. The number average molecular weight as determined by GPC is suitably between 200 and 65,000, most preferably between 200 and 17,500.

As used herein C₁₋₁₂-alkyl means a linear or branched alkyl group having 1 to 12 carbon atoms, C₁₋₈-alkyl means a linear or branched alkyl group having 1 to 8 carbon atoms, and C₁₋₆-alkyl means a linear or branched alkyl group having 1 to 6 carbon atoms, such as methyl, ethyl, propyl, isopropyl, butyl, pentyl and hexyl.

As used herein C₁₋₄-alkylene means a linear or branched divalent alkylene group having 1 to 4 carbon atoms, such as methylene, ethylene, propylene, isopropylene, butylenes and isobutylene.

As used herein C₃₋₈-cycloalkyl means a cyclic alkyl group having 3 to 8 carbon atoms, such as cyclopentyl and cyclohexyl.

As used herein C₆₋₁₄-aryl means a phenyl or naphthyl group optionally substituted with C₁₋₆- alkyl, such as tolyl and xylyl.

As used herein C₇₋₁₂-arylalkyl means aryl attached to a C₁₋₆-alkyl group, where C₁₋₆-alkyl and aryl is as defined above, such as benzyl, phenethyl and o-methylphenethyl.

In the compound of formula (II) and in the compound of formula (III), the groups R and R³, which are not hydrogen, are suitably each independently selected from a member of the group C₁₋₆-alkyl, C₆₋₁₄-aryl or C₇₋₁₂-arylalkyl.

The Si-H groups may be situated at either end of the compound of formula (II). However, at least one Si-H group is preferably positioned within the - (SiO(R³,R³))ₘ- chain of the compound of formula (II).

The polysiloxane cross-linking agent and the chain extender may be prepared as described in JP2004067720 International Publication No. WO 05/032401 or analogously to the methods described therein.

An addition reaction is, in its simplest terms, a chemical reaction in which the atoms of an element or compound react with a double bond or triple bond in an organic compound by opening up one of the bonds and becoming attached to it, forming one larger compound. Addition reactions are limited to chemical compounds that have multiple-bonded atoms. Hydrosilylation is an addition reaction between, for example, a carbon-carbon double bond in a compound and a reactive hydrogen from a hydrogen siloxane.

Suitable addition reaction catalysts are any hydrosilylation catalysts, preferably platinum (Pt) catalysts. Pt-catalysts for the first part of the two-component sealant are described in US Patent No. 6,248,915. In consideration of toxicity potential, Pt complex catalyst where Pt is at a valency state of zero is preferred. Preferred catalysts are platinum-vinylsiloxanes and platinum-olefin complexes, such as Pt-divinyl tetramethyl disiloxane.

The reaction is suitably carried out neat at a temperature between 25°C and 150 °C. It is not necessary to use a solvent for the reaction, which is an advantage for any adhesive, but especially for skin applications.

Suitably, the ratio of the number of reactive Si-H groups in the polysiloxane cross-linking agent to the number of unsaturated groups in the polypropylene oxide, which are reactive with Si-H groups under the reaction conditions, is between 0,2 and 1,0.

The amount of polysiloxane used for the cross-linking is suitably less than 15 % w/w and more preferred below 10 % w/w of the amount of polyalkylene oxide polymer having unsaturated end groups.

The cross-linking reaction does not lead to complete cross-linking of all the polyalkylene oxide polymers. The adhesive comprises a mixture of cross-linked and non cross-linked polyalkylene oxide polymers.

The additional second adhesive according to the invention may contain other conventional ingredients for adhesive compositions, such as tackifiers, extenders, non-reactive polymers, oils (e.g. polypropylenoxide, ethyleneoxide-propyleneoxide copolymers, mineral oil), plastizisers, fillers, and surfactants. The adhesive may also comprise pharmaceutically active ingredients. These optional ingredients may be present in the reaction mixture during the cross linking reaction.

It may be advantageous that the first adhesive comprises absorbent particles. According to an embodiment of the invention the first adhesive comprises absorbent particles.

The particles may be absorbent particles such as hydrocolloids, microcolloids or super absorbers in order for the composition to absorb moisture from skin.

Microcolloid particles are well-known in the art e.g. from International Publication No. WO 02/066087, which discloses adhesive compositions comprising microcolloid particles. The microcolloid particles may have a particle size of less than 20 microns.

The first adhesive may comprise 1 to 60 % w/w of hydrocolloid (HC) or super absorbent particles (SAP) particles, more preferred 30 to 60% w/w particles.

According to a preferred embodiment of the invention, the first adhesive comprises absorbent particles.

According to a preferred embodiment of the invention, the absorbent particles are selected from hydrocolloids, microcolloids and super absorbers.

In an embodiment of the invention, the first adhesive is located on the skin-facing surface of the additional second adhesive.

By the skin-facing surface of the adhesive is meant the side adhering to the skin.

By located is meant where the additional second adhesive is placed on the collecting device and specifies the position of the additional second adhesive or the first adhesive on the collecting device.

In another embodiment of the invention, the additional second adhesive is located on the pouch-facing surface of the adhesive wafer.

By the pouch-facing surface or non-skin-facing surface is meant the side of the adhesive or backing pointing away from the skin (non-bonding side).

According to one embodiment of the invention, the additional second adhesive is located at the outer rim of the adhesive wafer.

By the outer rim portion of the adhesive wafer is meant the portion essentially outside the welding zone or coupling. This portion is less exposed to faeces but has to handle perspiration as well as mechanical exposure due to movements and pull from the bag.

By the inner rim portion of the adhesive wafer is meant the portion in the perianal, peristomal or fistal area. This area of the adhesive is exposed to faeces or exudates as well as perspiration. The properties of the inner rim portion depend on wear time, output type and anatomy among others.

According to an embodiment of the invention, the additional second adhesive is in the form of a ring.

By an adhesive ring is meant an adhesive, essentially surrounding the body opening that needs to be drained or a ring covering the outer or inner rim of the adhesive wafer

According to one embodiment of the invention, the additional second adhesive is part of a ring such as a half ring covering 180 degrees of a circle.

According to an embodiment of the invention, the first adhesive only covers a part of the additional second adhesive on the skin-facing surface.

According to an embodiment of the invention, the additional second adhesive partly covers the adhesive wafer.

According to another embodiment of the invention, the additional second adhesive partly covers the skin-facing surface of the first adhesive.

According to another embodiment of the invention, the adhesive wafer has a recess for adapting the first adhesive.

The collecting device could be pre-shaped with a recess that fits the first adhesive to be built in the device. A recess could be a cut-out of part of the additional second adhesive.

By adapting is meant that the first adhesive is cast within the collecting device.

According to an embodiment of the invention, the device has an optional backing layer for the first adhesive.

Sealing an opening refers to protection of an opening from output, for example use of a paste around a stoma at the rim of the perineal skin or the rim of a fistula.

According to an embodiment of the invention, the body waste source is a stoma.

According to another embodiment of the invention, the body waste source is a fistula.

According to another embodiment of the invention, the body waste source is an anus.

By a body waste source is meant a natural or artificial body opening such as an anus, a stoma or a fistula.

According to an embodiment of the invention, the collecting device is an ostomy appliance.

According to another embodiment of the invention, the collecting device is a faecal collecting device.

According to another embodiment of the invention, the collecting device is a fistula collecting device.

According to an embodiment of the invention, the collecting pouch is detachable.

According to another embodiment of the invention, the collecting pouch is integrated with the wafer.

The collecting pouch may be detachable from the adhesive wafer by a coupling system or the pouch and the wafer may be integrated with the wafer, e.g. by welding. The two versions are known as one piece or two-piece appliances for ostomy.

### Description of the preferred embodiments

The invention is now explained more in detail with reference to the drawings showing preferred embodiments of the invention.

Figures 1 to 4 are all drawings of a side view of the left half of the adhesive wafer according to the invention. All wafers are covered with a backing layer. The skin-facing surface is illustrated in the drawings as the downward side of the wafer and the backing layer is placed on top of the wafer.

Figure 1 shows an adhesive wafer with a first adhesive (11) and an additional second adhesive (12), where the first adhesive only covers part of the adhesive wafer and is placed on the surface of the skin-facing adhesive wafer. The first adhesive can optionally be covered by a second backing layer placed in between the non-skin-facing side of the first adhesive and the skin-facing side of the additional second adhesive.

Figure 2 illustrates a side view of an adhesive wafer with a first adhesive (21) partly covered with an additional second adhesive (22) in the outer rim portion of the wafer.

Figure 3 illustrates a side view of another embodiment of the invention, where an adhesive wafer with an additional second adhesive (32) has a recess for adapting the first adhesive (31).

Figure 4 illustrates a side view of an embodiment of the invention, where an adhesive wafer with a first adhesive (41) is in combination with an additional second adhesive (42) in the inner portion of the adhesive wafer.

## Claims

1. A body waste collecting device comprising
- a collecting pouch
- an adhesive wafer for attachment to the body, comprising
- a backing layer
- a first adhesive (11,21,31 respectively 41)
- an additional second adhesive (12,22,32 respectively 42),
wherein said first adhesive is a hydrocolloid pressure sensitive adhesive and said additional second adhesive comprises a polyalkyleneoxide polymer and an organosiloxane based cross-linked adhesive system.

2. The collecting device according to claim 1, wherein the polyalkylene oxide polymer is polypropyleneoxide.

3. The collecting device according to any of the preceding claims, wherein the first adhesive (11,21,31 respectively 41) is located adjacent to the body waste source.

4. The collecting device according to any of claims 1 to 3, wherein the first adhesive (11 respectively 31) is located on the skin-facing surface of the additional second adhesive (12 respectively 32).

5. The collecting device according to any of claims 1 to 3, wherein the additional second adhesive (12,22,32 respectively 42) is located on the pouch-facing surface of the adhesive wafer.

6. The collecting device according to any of the preceding claims, wherein the additional second adhesive (12,22,32 respectively 42) is located at the outer rim of the adhesive wafer.

7. The collecting device according to any of the preceding claims, wherein the additional second adhesive (12,22,32 respectively 42) is in the form of a ring.

8. The collecting device according to any of the preceding claims, wherein the first adhesive (11,21 respectively 31) partly covers the additional second adhesive (12,22 respectively 32) on the skin-facing surface.

9. The collecting device according to any of the preceding claims, wherein the additional second adhesive (22 respectively 42) partly covers the adhesive wafer.

10. The collecting device according to any of the preceding claims, wherein the adhesive wafer has a recess for adapting the first adhesive (31).

11. The collecting device according to any of the preceding claims, wherein the device has an optional backing layer for the first adhesive (11,21,31 respectively 41).

12. The collecting device according to any of the preceding claims, wherein the collecting device is an ostomy appliance.

13. The collecting device according to any of the preceding claims, wherein the collecting device is a faecal collecting device.

14. The collecting device according to any of claims 1 to 11, wherein the collecting device is a fistula collecting device.

15. The collecting device according to any of the preceding claims, wherein the collecting pouch is detachable.

16. The collecting device according to any of the preceding claims, wherein the collecting pouch is integrated with the wafer.

## Patentansprüche

1. Vorrichtung zum Sammeln von Körperabfällen, umfassend
- einen Sammelbeutel
- eine Haftscheibe (adhesive wafer) zur Befestigung am Körper, umfassend
- eine Trägerschicht
- einen ersten Klebstoff (11, 21, 31 bzw. 41)
- einen zusätzlichen zweiten Klebstoff (12, 22, 32 bzw. 42),
wobei der erste Klebstoff ein Hydrokolloid-Haftklebstoff ist und der zusätzliche zweite Klebstoff ein Polyalkylenoxid-Polymer und ein vernetztes Klebstoffsystem auf Organosiloxanbasis umfasst.

2. Sammelvorrichtung gemäß Anspruch 1, wobei das Polyalkylenoxid-Polymer Polypropylenoxid ist.

3. Sammelvorrichtung gemäß einem der vorstehenden Ansprüche, wobei der erste Klebstoff (11, 21, 31 bzw. 41) benachbart zu der Körperabfall-Quelle angeordnet ist.

4. Sammelvorrichtung gemäß einem der Ansprüche 1 bis 3, wobei der erste Klebstoff (11 bzw. 31) an der zu der Haut gerichteten Oberfläche des zusätzlichen zweiten Klebstoffs (12 bzw. 32) angeordnet ist.

5. Sammelvorrichtung gemäß einem der Ansprüche 1 bis 3, wobei der zusätzliche zweite Klebstoff (12, 22, 32 bzw. 42) an der zu dem Beutel gerichteten Oberfläche der Haftscheibe angeordnet ist.

6. Sammelvorrichtung gemäß einem der vorstehenden Ansprüche, wobei der zusätzliche zweite Klebstoff (12, 22, 32 bzw. 42) an dem äußeren Rand der Haftscheibe angeordnet ist.

7. Sammelvorrichtung gemäß einem der vorstehenden Ansprüche, wobei der zusätzliche zweite Klebstoff (12, 22, 32 bzw. 42) in der Form eines Rings vorliegt.

8. Sammelvorrichtung gemäß einem der vorstehenden Ansprüche, wobei der erste Klebstoff (11, 21 bzw. 31) den zusätzlichen zweiten Klebstoff (12, 22 bzw. 32) an der zu der Haut gerichteten Oberfläche teilweise bedeckt.

9. Sammelvorrichtung gemäß einem der vorstehenden Ansprüche, wobei der zusätzliche zweite Klebstoff (22 bzw. 42) die Haftscheibe teilweise bedeckt.

10. Sammelvorrichtung gemäß einem der vorstehenden Ansprüche, wobei die Haftscheibe eine Vertiefung zum Aufnehmen des ersten Klebstoffs (31) aufweist.

11. Sammelvorrichtung gemäß einem der vorstehenden Ansprüche, wobei die Vorrichtung eine optionale Trägerschicht für den ersten Klebstoff (11, 21, 31 bzw. 41) aufweist.

12. Sammelvorrichtung gemäß einem der vorstehenden Ansprüche, wobei die Sammelvorrichtung eine Stoma-Anwendung ist.

13. Sammelvorrichtung gemäß einem der vorstehenden Ansprüche, wobei die Sammelvorrichtung eine Kotsammelvorrichtung ist.

14. Sammelvorrichtung gemäß einem der Ansprüche 1 bis 11, wobei die Sammelvorrichtung eine Fistel-Sammelvorrichtung ist.

15. Sammelvorrichtung gemäß einem der vorstehenden Ansprüche, wobei der Sammelbeutel ablösbar ist.

16. Sammelvorrichtung gemäß einem der vorstehenden Ansprüche, wobei der Sammelbeutel mit der Scheibe integriert ist.

## Revendications

1. Dispositif de collecte de déchets corporels comprenant
- une poche de collecte
- un gaufrage adhésif pour fixation au corps, comprenant
- une couche de support
- un premier adhésif (11, 21, 31 respectivement 41)
- un deuxième adhésif additionnel (12, 22, 32 respectivement 42),
où ledit premier adhésif est un adhésif autocollant d'hydrocolloïde et ledit deuxième adhésif additionnel comprend un polymère de poly(oxyde d'alkylène) et un système adhésif réticulé à base d'organosiloxane.

2. Dispositif de collecte selon la revendication 1, dans lequel le polymère de poly(oxyde d'alkylène) est un poly(oxyde de propylène).

3. Dispositif de collecte selon l'une quelconque des revendications précédentes, dans lequel le premier adhésif (11, 21, 31 respectivement 41) est situé en position adjacente à la source de déchets corporels.

4. Dispositif de collecte selon l'une quelconque des revendications 1 à 3, dans lequel le premier adhésif (11 respectivement 31) est situé sur la surface côté peau du deuxième adhésif additionnel (12 respectivement 32).

5. Dispositif de collecte selon l'une quelconque des revendications 1 à 3, dans lequel le deuxième adhésif additionnel (12, 22, 32 respectivement 42) est situé sur la surface côté poche du gaufrage adhésif.

6. Dispositif de collecte selon l'une quelconque des revendications précédentes, dans lequel le deuxième adhésif additionnel (12, 22, 32 respectivement 42) est situé au niveau du rebord extérieur du gaufrage adhésif.

7. Dispositif de collecte selon l'une quelconque des revendications précédentes, dans lequel le deuxième adhésif additionnel (12, 22, 32 respectivement 42) est sous la forme d'un anneau.

8. Dispositif de collecte selon l'une quelconque des revendications précédentes, dans lequel le premier adhésif (11, 21 respectivement 31) recouvre partiellement le deuxième adhésif additionnel (12, 22 respectivement 32) sur la surface côté peau.

9. Dispositif de collecte selon l'une quelconque des revendications précédentes, dans lequel le deuxième adhésif additionnel (22 respectivement 42) recouvre partiellement le gaufrage adhésif.

10. Dispositif de collecte selon l'une quelconque des revendications précédentes, dans lequel le gaufrage adhésif comporte un évidement pour adapter le premier adhésif (31).

11. Dispositif de collecte selon l'une quelconque des revendications précédentes, dans lequel le dispositif a une couche de support facultative pour le premier adhésif (11, 21, 31 respectivement 41).

12. Dispositif de collecte selon l'une quelconque des revendications précédentes, dans lequel le dispositif de collecte est un dispositif de stomie.

13. Dispositif de collecte selon l'une quelconque des revendications précédentes, où le dispositif de collecte est un dispositif de collecte fécal.

14. Dispositif de collecte selon l'une quelconque des revendications 1 à 11, où le dispositif de collecte est un dispositif de collecte de fistule.

15. Dispositif de collecte selon l'une quelconque des revendications précédentes, dans lequel la poche de collecte est détachable.

16. Dispositif de collecte selon l'une quelconque des revendications précédentes, dans lequel la poche de collecte est intégrée au gaufrage.
